# FASCICULE DE BREVET EUROPEEN

(11) **EP 2 937 064 B1**
(45) Date de publication et mention de la délivrance du brevet: **24.08.2016**
(21) Numéro de dépôt: 15164936.5
(22) Date de dépôt: 22.04.2011
(51) Int. Cl.: A61F 2/00

(54) **PROCÉDÉ DE FABRICATION D'UN IMPLANT DE SOUTIEN DE L'URÈTRE POUR LE TRAITEMENT DE L'INCONTINENCE URINAIRE MASCULINE**
HERSTELLUNGSVERFAHREN EINES IMPLANTATS ZUR STÜTZUNG DER HARNRÖHRE ZUR BEHANDLUNG VON HARNINKONTINENZ BEI MÄNNERN
METHOD FOR MANUFACTURING A URETHRA-SUPPORTING IMPLANT FOR THE TREATMENT OF MALE URINARY INCONTINENCE

(30) Priorité: 23.04.2010 FR 1053091
(43) Date de publication de la demande: 28.10.2015
(62) Demande divisionnaire de: 11723499.7
(73) Titulaire: Aspide Medical, 42350 La Talaudiere (FR); Le Portz, Benoît, 56000 Vannes (FR)
(72) Inventeur: Le Portz, Benoît, 56000 Vannes (FR)
(74) Mandataire: Cabinet Laurent & Charras

(56) Documents cités:
- WO-A1-2009/086446
- WO-A2-2007/149348
- WO-A2-2009/086369
- FR-A1- 2 906 131
- US-A1- 2004 039 453

## Description

L'invention se rattache au secteur technique des implants permettant le traitement de l'incontinence urinaire chez l'homme. Cette incontinence peut résulter d'une procédure de prostatectomie pratiquée pour le traitement du cancer de la prostate.

De manière plus générale, il existe des implants ou dispositifs pour traiter cette incontinence qui reprennent majoritairement le principe de la bandelette sous-urétrale, bien connu pour le traitement de l'incontinence urinaire d'effort chez la femme.

De nombreux implants ou dispositifs ont ainsi été décrits pour traiter l'incontinence de l'homme ou de la femme. Par exemple, le Demandeur a développé des solutions décrites dans les brevets français 2934150, 2926455. D'autres publications sont survenues par des tiers tels que FR 2906131, US 7431690, US 7559885, EP 2025304.

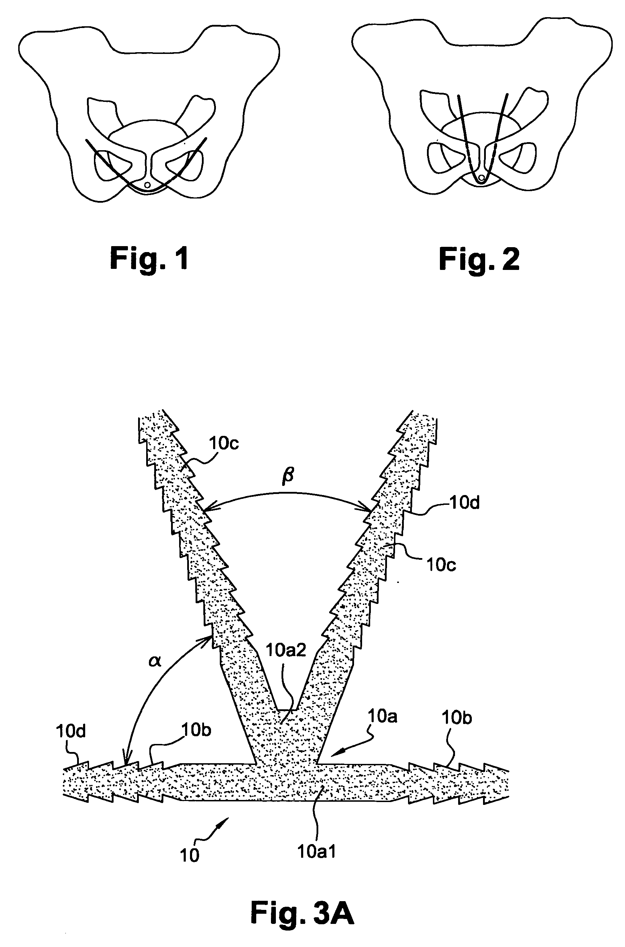

Dans le cadre du traitement de l'incontinence urinaire chez la femme les concepts principaux utilisés font que la bandelette est insérée à travers les trous obturateurs du bassin selon un passage trans-obturateur. Il est proposé aussi que la bandelette soit insérée en arrière du pubis dans le cadre d'un passage dénommé rétro-pubien. On a ainsi illustré de manière schématique aux figures 1 et 2 le passage de la bandelette dans les deux situations de pose précitées

Pour l'incontinence urinaire de l'homme, de sévérité mineure à sévérité modérée, les dispositifs sont majoritairement basés sur le passage trans-obturateur.

Le Demandeur a constaté que les dispositifs ou implants ainsi utilisés restaient peu performants. En effet, les solutions apportées par l'Art Antérieur assurent un soutien de l'urètre localisé et cela peut entraîner une migration de la bandelette ou une efficacité limitée.

On connaît par ailleurs par les documents US 2004/039453, WO 2009/086446 et WO-A-2007/149348 des implants dont les parties constitutives ont des caractéristiques et propriétés mécaniques pouvant varier par des procédés de tissage / tricotage différents.

La démarche du Demandeur a donc été de reconsidérer la conception de l'implant pour permettre une meilleure tenue de l'urètre et limiter le risque de migration de la bandelette et d'apporter une optimisation. La solution apportée par le Demandeur en considérant l'Art Antérieur est de conception simple et répond avantageusement à la problématique.

L'invention concerne un procédé de fabrication d'un implant de l'urètre selon la revendication 1.

Ces caractéristiques et bien d'autres encore ressortiront bien de la suite de la description.

Pour fixer l'objet de l'invention illustré d'une manière non limitative aux figures des dessins où seulement les figures 10, 11 et 13 montrent un procédé selçn l'invention.
Les figures 1 et 2 sont des vues illustrant de manière schématique des implants utilisés pour l'incontinence de la femme sous forme de bandelette et se positionnant par rapport au bassin selon respectivement un passage trans-obturateur et un passage rétro-pubien.
Les figures 3A, 3B, 3C, 3D, 3E sont des vues à caractère schématique illustrant l'implant de soutien de l'urètre,
La figure 4 est une vue d'un implant de soutien dans sa présentation globale,
La figure 5 est une vue illustrant la pose de l'implant de soutien et son passage et positionnement par rapport au bassin, un procédé selon l'invention.
La figure 6 est une vue à caractère schématique de l'implant de soutien en situation,
La figure 7 est une vue à caractère schématique illustrant un rouleau de tricot à partir duquel est fabriqué l'implant dans une version optimisée de l'invention,
La figure 8A est une vue à plat à caractère schématique illustrant dans la version optimisée en première variante la découpe des bras trans-obturateurs et pré-pubiens dans un sens longitudinal du tricot et aussi de la partie centrale,
La figure 8B est une vue à plat de l'implant assemblé tel que découpé selon la figure 8A,
La figure 9A est une vue à plat à caractère schématique illustrant dans la version optimisée dans une seconde variante la découpe monobloc des bras trans-obturateurs avec la partie centrale et la découpe des bras pré-pubiens indépendants dans le sens longitudinal du tricot,
La figure 9B est une vue à plat de l'implant assemblé tel que découpé selon la figure 9A,
La figure 10A est une vue à plat à caractère schématique illustrant dans la version optimisée une troisième variante la découpe monobloc des bras pré-pubiens avec la partie centrale et la découpe des bras trans-obturateurs dans le sens longitudinal du tricot,
La figure 10B est une vue à plat de l'implant assemblé tel que découpé selon la figure 10A,
La figure 11A est une vue à plat à caractère schématique illustrant la découpe monobloc des bras pré-pubiens avec la partie centrale et la découpe indépendante et monobloc des bras trans-obturateurs dans le sens longitudinal du tricot,
La figure 11B est une vue à plat de l'implant assemblé tel que découpé selon la figure 11A,
La figure 12 est une vue en variante de la découpe des composants par exemple ceux de la figure 8A dans le sens transversal du tricot,
Les figures 13A et 13B sont des vues à caractère schématique illustrant le moyen d'assemblage des bras trans-obturateurs par couture respectivement en zig-zag, et droite, et sans contrainte,
La figure 13C est une vue de l'implant selon la figure 13A avec une traction appliquée sur les bras trans-obturateurs

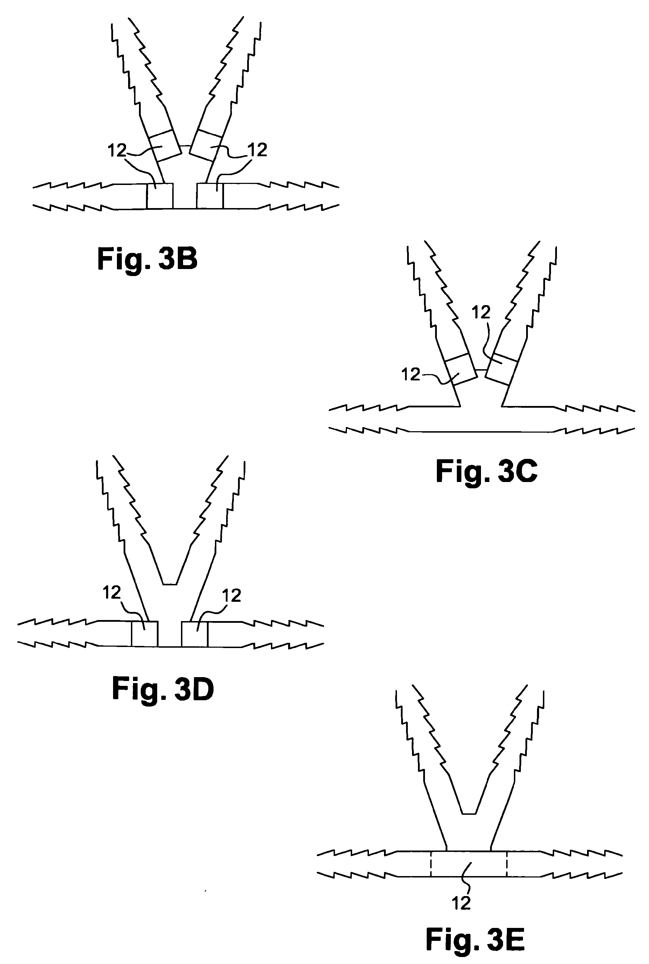

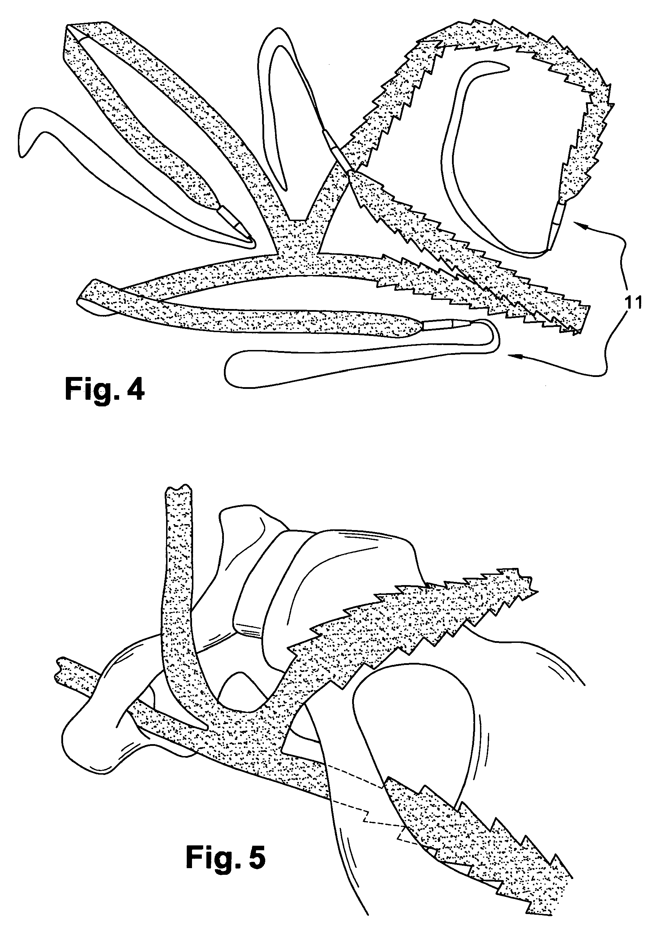

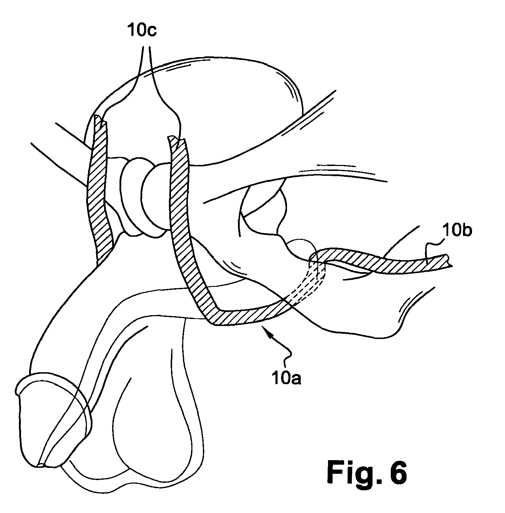

En se référant aux figures 3 à 13C, l'implant de soutien de l'urètre pour le traitement de l'incontinence urinaire masculine est du type réalisé sous la forme d'une bandelette (10), tricotée, tressée ou autres, en matériau polypropylène ou similaire biocompatible pour être posée sous l'urètre du patient. Elle est réalisée à partir d'un rouleau (R) de tricot apte à cette application.

L'implant sous forme de bandelette comprend une partie centrale (10a) destinée à constituer la zone de soutien de l'urètre. Cette partie centrale présente une base inférieure (10a1) susceptible de se prolonger pour permettre la fixation et la liaison dans un même plan et en opposition de deux bras (10b) trans-obturateurs. La longueur de ces bras est établie selon l'anatomie du bassin masculin. La base inférieure (10a1) se prolonge par le haut par une section en débordement (10a2) de la dite base inférieure et à partir de laquelle sont disposés et fixés deux bras (10c) pré-pubiens. Ces derniers sont dans une configuration en 'V' l'un par rapport à l'autre selon une amplitude et angulation tenant compte de la morphologie du bassin. Cette angulation β est de l'ordre de 30 à 50°. Par ailleurs chacun des dits bras pré-pubiens (10C) est également dans une configuration en 'V' par rapport aux bras sous-jacents (10B) trans-obturateurs selon une amplitude ou angulation α de l'ordre de 60 à 80°.

L'implant ainsi réalisé peut former un tout monobloc et dans le même matériau (figure 3A) ou bien les parties de bras (10b)-(10c) peuvent être avantageusement reliées à la partie centrale de toute manière appropriée et notamment par soudure, couture ou autres (12) (figures 3B, 3C, 3D, 3E), ceci afin de pouvoir optimiser leurs caractéristiques mécaniques, notamment l'allongement, et choisir ainsi le meilleur sens du tricot constituant chaque bras (10b)-(10c)..

Dans une variante, au moins un des bras de prolongement (10b) (10c) est réalisé d'un seul bloc avec la partie centrale (10a) et au moins un des bras de prolongement (10b) (10c) est réalisé de façon indépendante et relié à la partie centrale (10a) par tous moyens.

Dans une variante, plusieurs bras de prolongement (10b) (10c) sont jointifs et reliés à la partie centrale (10a) par tous moyens (figure 3E).

Dans une mise en oeuvre optimisée, les bras de prolongement (10b-10c) sont réalisés en un même matériau que la partie centrale (10a)

Dans cette mise en oeuvre optimisée, on considère le rouleau de tricot (R) dans lequel on va découper les différentes parties constitutives de l'implant afin d'avoir et d'obtenir la découpe des dits bras pré-pubiens et trans-obturateurs dans le sens le plus résistant du tricot.

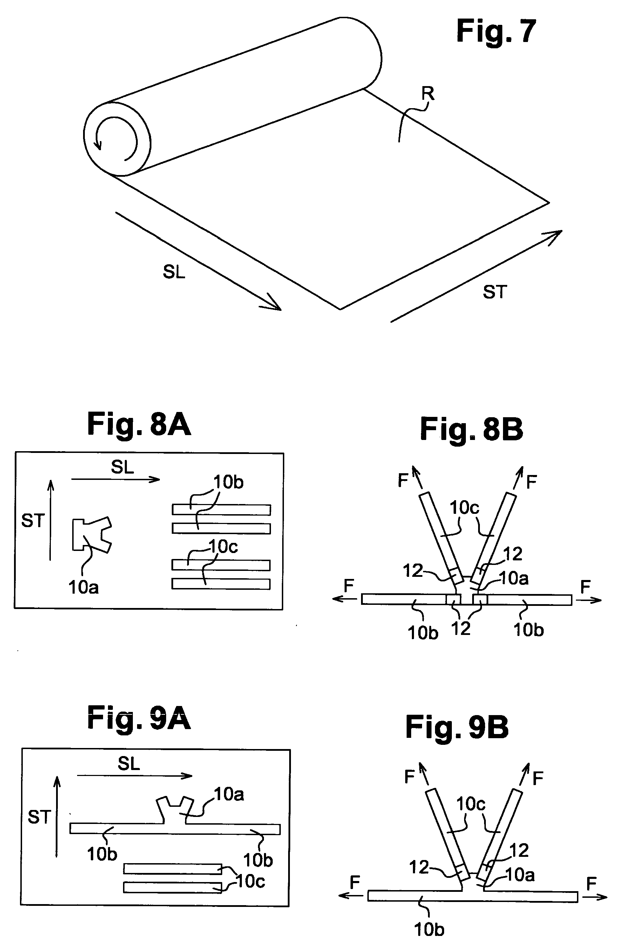

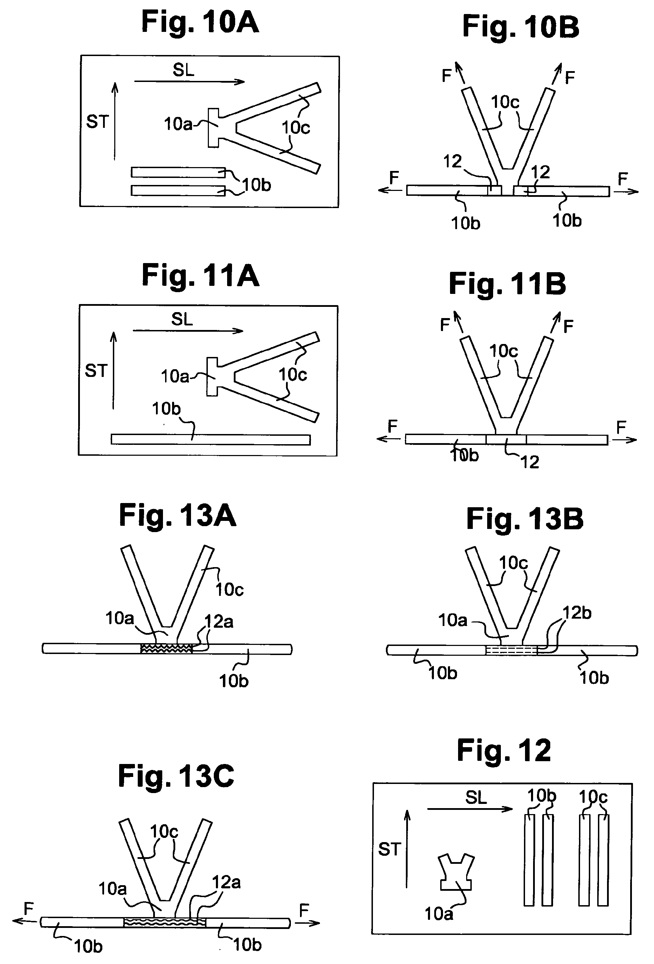

Ce dernier a un sens de déroulement, et le sens longitudinal (SL) est défini comme étant le sens de déroulement du tricot, et le sens transversal (ST), celui qui est perpendiculaire au sens longitudinal, figure 7. Le tricot a ainsi un sens plus résistant mécaniquement que l'autre et un sens à capacité plus extensible que l'autre. Les propriétés peuvent ainsi être établies conformément aux figures 8A à 11B où le sens longitudinal présente la propriété d'être plus résistant mécaniquement, et le sens transversal plus extensible. A l'inverse, figure 12, c'est le sens transversal qui est le plus résistant mécaniquement et le sens longitudinal le plus extensible (ceci dépendant de la façon dont est réalisé le tricot). L'essentiel selon l'invention réside dans le fait que les bras trans-obturateurs et les bras pré-pubiens soient tous découpés dans le même sens. Ainsi, afin d'optimiser la constitution de l'implant et notamment le comportement mécanique de ses bras, l'invention consiste à découper chaque partie de l'implant et donc les bras dans le sens et orientation de manière la plus favorable du tricot en terme de comportement mécanique (résistance mécanique maximale et allongement minimal). Les parties sont ensuite assemblées pour constituer l'implant final.

En se référant aux figures 8A et 8B, les quatre bras trans-obturateurs (10b) et pré-pubiens (10c) sont tous découpés dans le sens longitudinal, et la partie centrale est découpée de sorte que ses parties de liaison avec les bras pré-pubiens soient dans l'orientation du sens longitudinal du tricot. Les parties d'assemblage sont identifiées par (12).

En se référant aux figures 9A-9B, les bras trans-obturateurs (10b) réalisés monoblocs avec la partie centrale (10a), ainsi que les bras pré-pubiens (10c) sont découpés dans le sens longitudinal du tricot. Les parties d'assemblage sont identifiées par (12).

En se référant aux figures 10A-10B, les bras pré-pubiens (10c) et la partie centrale (10a) sont découpés monoblocs, tandis que les bras trans-obturateurs (10b) sont indépendants. Les bras pré-pubiens sont orientés dans le sens longitudinal du tricot mais selon une orientation angulaire tout en conservant en grande partie les propriétés du tricot dans le sens longitudinal. Les parties d'assemblage sont identifiées par (12).

En se référant aux figures 11A-11B, les bras pré-pubiens (10c) sont monoblocs avec la partie centrale (10a), et les bras trans-obturateurs (10b) sont réalisés en un ensemble monobloc découpé dans le tricot. Les bras pré-pubiens sont orientés dans le sens longitudinal du tricot mais selon une orientation angulaire tout en conservant en grande partie les propriétés du tricot dans le sens longitudinal. Les bras trans-obturateurs sont orientés dans le sens longitudinal du tricot.

On a représenté à titre schématique figure 12 la découpe des différents composants telle qu'exposée aux figures 8A à 11A dans le sens transversal du tricot dans le cadre d'une optimisation des caractéristiques de chaque bras (10b-10c) en fonction de la maille du rouleau de tricot à découper. Bien évidemment, les différentes variantes de découpe des figures 8A à 11 A s'appliquent à une découpe dans le sens transversal du tricot.

Les solutions de découpe ainsi utilisées permettent donc la découpe de chaque bras (10b-10c) dans le sens le plus favorable du tricot selon l'orientation de la maille de celui-ci, et donc à une optimisation et homogénéité des caractéristiques mécaniques de chaque bras (résistance mécanique maximale et allongement minimal lorsque les bras sont soumis à une force de traction (F).

En outre, selon les variantes de la découpe, cela permet de limiter le nombre de composant à découper et à assembler avec une optimisation de la production.

Dans le cas de la mise en oeuvre d'un ensemble monobloc et continu des bras trans-obturateurs (10b), on attend un assemblage encore plus résistant.

La liaison de chaque bras avec la partie centrale précitée est établie de toute manière appropriée, et la partie centrale présentant dans tous les cas une amorce de configuration et de positionnement des bras.

Le ou les bras peuvent présenter avantageusement comme illustrés d'une manière non limitative aux dessins, des formes en harpons (10d) ou crans susceptibles de constituer des points d'ancrage afin de limiter le glissement des bras dans les tissus. En extrémité des dits bras peuvent être prévues des boucles (11) ou embouts profilés ou tout autre moyen de préhension pour faciliter le passage des bras.

Selon une autre disposition avantageuse représentée aux figures 13A à 13C, on a représenté un implant réalisé en deux parties assemblées et comprenant une pièce monobloc avec bras pré-pubiens (10c) et partie centrale (10a), et une autre pièce monobloc constituée par les bras trans-obturateurs (10b) formant une bande continue unique.

L'assemblage et liaison s'effectue par une couture en zig-zag (12a), figure 13A, ou une couture droite (12b), figure 13B.

L'intérêt de la couture en zig-zag comme il apparaît figure 13C est de pouvoir absorber plus facilement les déformations et contraintes mécaniques lors de la mise en tension des bras trans-obturateurs sans risque de rupture précoce du fil de couture.

L'implant ainsi réalisé offre une double stabilité, trans-obturatrice d'une part et pré-pubienne, d'autre part. Les couples de bras sont mis en place de manière connue à l'aide d'ancillaires adaptés à chaque passage. L'implant offre par sa partie centrale une zone de soutien très conséquente de l'urètre qui est ainsi maintenue en position tractée par le biais des quatre bras précités, traitant ainsi l'incontinence urinaire de façon efficace chez l'homme.

Selon l'invention, la partie centrale et les quatre bras sont réalisés et découpés à partir du même matériau et d'un même tricot. La découpe des quatre bras dans le même sens du tricot longitudinal ou transversal et avec les mêmes propriétés mécaniques optimales permet à la fois d'optimiser les caractéristiques mécaniques et d'allongement des bras, et leur orientation afin de créer des bras trans-obturateurs et pré-pubiens efficaces

L'invention est ainsi remarquable par le procédé de découpe de l'implant ainsi défini dans un tricot, dans un sens longitudinal ou transversal de l'ensemble des composants de l'implant en vue d'avoir pour les bras les mêmes propriétés mécaniques dans un souci d'homogénéité et d'optimisation de l'utilisation de l'implant. Le tricot qui présente deux sens, longitudinal et transversal, a toujours un sens plus résistant que l'autre, et l'originalité de l'invention est de découper les bras (10b) (10c) de l'implant dans le sens le plus résistant mécaniquement.

## Revendications

1. Procédé de fabrication d'un implant de soutien de l'urètre pour le traitement de l'incontinence urinaire masculine, de type bandelette réalisée en un matériau bio-compatible du type comprenant une partie centrale (10a) destinée à constituer la zone de soutien de l'urètre, comprenant une base inférieure (10a1) se prolongeant sous la forme de deux bras trans-obturateurs (10b) dans un même plan et en opposition, la base inférieure étant prolongée par le haut par une section en débordement (10a2) de la dite base inférieure, cette section en débordement (10a2) se prolongeant également sous la forme de deux bras pré-pubiens (10c) ; les dits bras pré-pubiens étant dans une configuration en 'V' l'un par rapport à l'autre, et chacun des dits bras pré-pubiens étant dans une configuration en V par rapport aux bras trans-obturateurs sous-jacents, les bras de prolongement (10b) (10c) étant réalisés en un même matériau que la partie centrale (10a), ledit procédé étant **caractérisé en ce qu'**il consiste à découper à partir d'un tricot les différentes parties constitutives de l'implant défini par les bras pré-pubiens (10c), les bras trans-obturateurs (10b) et la partie centrale (10a), les bras pré-pubiens (10c) sont monoblocs avec la partie centrale (10a), **caractérisé en ce que** les bras trans-obturateurs (10b) sont découpés, les bras pré-pubiens sont orientés dans le sens longitudinal du tricot mais selon une orientation angulaire tout en conservant en grande partie les propriétés du tricot dans le sens longitudinal, les bras trans-obturateurs sont orientés dans le sens longitudinal du tricot et **en ce que** la découpe des bras est effectuée dans le sens le plus résistant du tricot, on assemble les bras trans-obturateurs par rapport à la partie centrale.

2. Procédé selon la revendication 1 **caractérisé en ce que** les bras trans-obturateurs sont indépendants.

3. Procédé selon la revendication 1 **caractérisé en ce que** les bras trans-obturateurs sont réalisés en un ensemble monobloc découpé dans le tricot.

4. Procédé selon la revendication 3 **caractérisé en ce que** l'on assemble par une couture en zig-zag les bras trans-obturateurs par rapport à la partie centrale.

## Patentansprüche

1. Verfahren zur Herstellung eines Stützimplantats für die Harnröhre zur Behandlung der Harninkontinenz bei Männern, in Form eines Bandes aus einem bio- kompatiblen Material mit einem Zentralteil (10a), der den Stützbereich der Harnröhre bilden soll, mit einer unteren Basis (10a1), die sich in Form von zwei trans- obturatorischen Armen (10b) in derselben Ebene und einander gegenüberliegend verlängert, die untere Basis wird nach oben durch einen überstehenden Abschnitt (10a2) dieser unteren Basis verlängert, der sich ebenfalls in Form von zwei prä- pubischen Armen (10c) verlängern; diese gennanten präpubischen Arme befinden sich in einer "V"- Konfiguration zueinander und jeder dieser prä- pubischen Arme befindet sich in einer "V"- Konfiguration bezogen auf die darunterliegenden trans- obturatorischen Arme, die Verlängerungsarme (10b) - (10c) sind aus demselben Material gefertigt wie der Zentralteil (10a), dieses Verfahren ist **dadurch gekennzeichnet, dass** es darin besteht aus einem Gewirk die verschiedenen Teile, aus denen das Implantat besteht, definiert durch die präpubischen Arme (10c), die trans- obturatorischen Arme (10b) und den Zentralteil (10a), zu schneiden, die prä- pubischen Armen (10c) bilden einen Einheit mit dem Zentralteil (10a), **dadurch gekennzeichnet, dass** die trans- obturatorischen Arme (10b) geschnitten werden, die präpubische Armen sind in einer Längsrichtung des Gewirks angeordnet, jedoch in einer Winkelausrichtung, wobei sie zum großen Teil die Eigenschaften des Gewirks in Längsrichtung bewahren, und die transobturatorischen Arme sind in der Längsrichtung des Gewirks ausgerichtet und dadurch dass das Schneiden der Arme in der widerstandsfähigsten Richtung des Gewirks erfolgt, die trans- obturatorischen Arme werden bezogen auf den Zentralteil verbunden.

2. Verfahren gemäß Anspruch 1, **dadurch gekennzeichnet, dass** die transobturatorischen Arme unabhängig sind.

3. Verfahren gemäß Anspruch 1, **dadurch gekennzeichnet, dass** die transobturatorischen Arme in einem zusammenhängenden Stück aus dem Gewirk geschnitten werden.

4. Verfahren gemäß Anspruch 3, **dadurch gekennzeichnet, dass** die transobturatorischen Arme bezogen auf den Zentralteil durch eine Zick-Zack-Naht verbunden werden.

## Claims

1. Manufacturing process for a urethral support sling implant for the treatment of male urinary incontinence, produced in a biocompatible material, comprising a central part (10a) intended to form the area supporting the urethra, comprising of an inferior base (10a1) prolonged in a same plane by two opposite trans-obturator arms (10b), the inferior base being prolonged upwards by a section (10a2) extending beyond the said inferior base, this section (10a2) also extending in the form of two pre-pubic arms (10c); the said pre-pubic arms being configured in a 'V' relative to each other, and each of the said pre-pubic arms being configured in a 'V' relative to the subjacent trans-obturator arms, the prolonging arms (10b) (10c) being made of the same material as the central part (10a), said process being **characterized in that** it consists of cutting the different parts which make up the implant, i.e. the pre-pubic arms (10c), the trans-obturator arms (10b) and the central part (10a), from a knitted fabric, the pre-pubic arms (10c) being monobloc with the central part (10c) **characterized in that** the trans-obturator arms (10b) are cut, the said pre-pubic arms (10c) are oriented in the longitudinal direction of the knitted fabric with an angular orientation while to a large extent retaining the longitudinal directional properties of the knitted fabric, the trans-obturator arms are oriented in the longitudinal direction of the knitted fabric, and the arms being cut out in the stronger direction of the knit, the trans-obturator arms being assembled with the central part.

2. Process according to claim 1 **characterized in that** the trans-obturator arms are independent.

3. Process according to claim 1 **characterized in that** the trans-obturator arms are made as a monobloc assembly cut in the knitted fabric.

4. Process according to claim 3 **characterized in that** the trans-obturator arms are assembled with the central part by a zig-zag sewing.
